# EUROPEAN PATENT SPECIFICATION

(11) **EP 0 456 826 B1**
(45) Date of publication and mention of the grant of the patent: **07.01.1999**
(21) Application number: 90914758.9
(22) Date of filing: 04.10.1990
(51) Int. Cl.: C07D 213/36, C07D 213/40, C07D 213/60, C07D 417/12, A01N 43/40, A01N 43/36, A01N 43/48, A01N 43/78

(54) **AMINE DERIVATIVES**
AMINDERIVATE
DERIVES D'AMINE

(30) Priority: 06.10.1989 JP 259966/89; 27.12.1989 JP 336231/89; 09.03.1990 JP 56611/90; 02.05.1990 JP 115246/90; 26.07.1990 JP 196258/90
(43) Date of publication of application: 21.11.1991
(73) Proprietor: NIPPON SODA CO., LTD., Chiyoda-ku, Tokyo 100-8165 (JP)
(72) Inventor: ISHIMITSU, Keiichi Odawara Research Center, Odawara-shi Kanagawa 250-02 (JP); SUZUKI, Junji Odawara Research Center, Takada Odawara-shi Kanagawa 250-02 (JP); OHISHI, Haruhito Odawara Research Center, Takada Odawara-shi Kanagawa 250-02 (JP); YAMADA, Tomio Odawara Research Center, Takada Odawara-shi Kanagawa 250-02 (JP); HATANO, Renpei Odawara Research Center, Takada Odawara-shii Kanagawa 250-02 (JP); TAKAKUSA, Nobuo Odawara Research Center, Takada Odawara-shi Kanagawa 250-02 (JP); MITSUI, Jun Odawara Research Center, Takada Odawara-shi KKanagawa 250-02 (JP)
(74) Representative: de Bruijn, Leendert C.
(86) International application number: JP9001282
(87) International publication number: WO9104965

(56) References cited:
- EP-A- 302 389
- EP-A- 302 833
- EP-A- 303 570
- EP-A- 306 696
- Chemical Abstracts, volume 88, no. 1, 2 January 1978, (Columbus, Ohio, US), Kreutzberger, Alfred : "Antimycotics. VI. Dehydro-N-Mannich bases from cyclicand mixed aliphatic-aromatic amines. ", see page 580, abstract 6816j, & Chem.-Ztg. 1977, 101( 9), 400- 40
- Chemical Abstracts, volume 94, no. 13, 30 March 1981, (Columbus, Ohio, US), see page 691, abstract 102861q, & JP, A, 80130950 (B-Alkoxyacrylonitriles, 3- aminoacrylonitriles, and 2-cyanovinyl esters.) 29 March 1979

## Description

### Technical Field:

The present invention relates to new amine derivatives, the processes for the production thereof and insecticides containing the said derivatives as effective compounds.

### Background art:

A large number of chemicals, for example, organophosphorus insecticides such as parathion and malathion and carbamate insecticides such as carbaryl and methomyl, have been developed and put to practical use by research and development on insecticides over many years. These insecticedes have played a very great role for the improvement of agricultural production. However, in recent years some of these insecticides are regulated on their use because of problems such as environmental pollution due to residue or accumulation, or cause infestitation of resistant insect pests as a result of long-term use. Therefore, it is demanded to develop new chemicals which have excellent insecticidal characteristics over various types of insect pests including these resistant insect pests and which can be used safely.

The following compound is known as the analogous compound of this invention, which has no insecticidal activity. 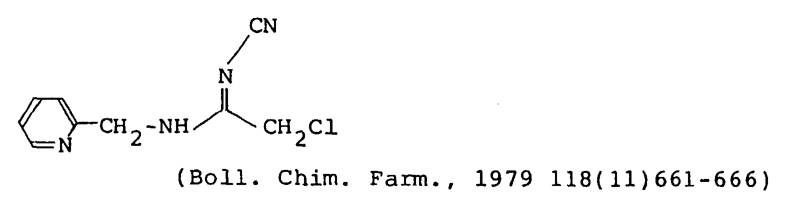 Further, the following compound is described in USP 4918088, which has insecticidal activities. 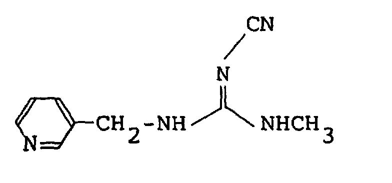 The compound however shows no insecticidal activity against lepidopterous insects and green rice leafhopper which are more serious pests on crops, though it shows the activity against cotton aphid.

EP-A-0 302 833 discloses i-nitro-2,2-diaminoethylene derivatives of the formula 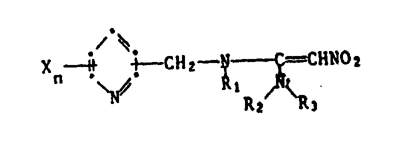 in which R₁ and R₂ are hydrogen or (cyclo)alkyl, R₃ is hydrogen (cyclo)alkyl, benzyl or pyridinylmethyl and X is a.o. halogen and alkoxy as insecticides.

EP-A-0 303 570 describes insecticides of the formula 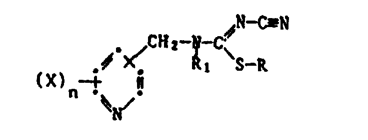 in which R is alkyl or benzyl, R₁ is alkyl, X is halogen and n = 0-3.

EP-A-0 306 696 discloses substituted N-pyridyl-N'-cyanoguanidine derivatives of the formula 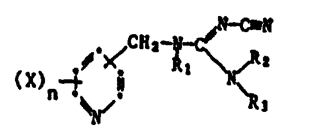 in which R₁ and R₃ are hydrogen or alkyl, R₂ is hydrogen, alkyl, benzyl or picolyl, X is halogen and n=0-3 as insecticides.

The purpose of this invention is to provide agricultural chemicals which can be advantageously synthesized industrially, have certain effects and are applicable safely.

The compound of this invention has high insecticidal activity against both lepidopterous and hemipterous insects.

### Disclosure of Invention:

The present invention relates to a compound having the formula 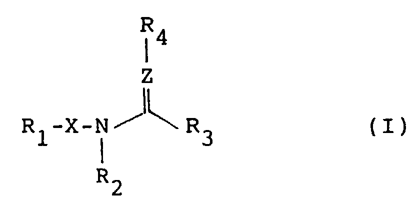 wherein in each case 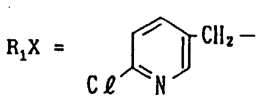 Z = N and R₄ = CN and wherein
- R₂ = H and R₃ = CH₂ CL ; or

- R₂ = H and R₃ = CH₂F; or
- R₂ = CH₃ and R₃ = H ; or
- R₂ = CH₃ and R₃ = CH₃ ; or
- R₂ = CH₃ and R₃ = CH₃ HCL salt ; or
- R₂ = CH₃ and R₃ = CH₂CL; or

- R₂ = CH₃ and R₃ = CH₂F; or
- R₂ = CH₃ and R₃ = C₂H₅ ; or
- R₂ = CH₃ and 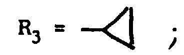 or
- R₂ = CH₃ and R₃ = CH₂OCH₃ or;

- R₂ = CH₃ and R₃ - CH₂SCH₃; or
- R₂ = CH₃ and R₃ = CH₂ COOC₂H₅
- R₂ = CH₃ and R₃ = CH₂CH₂CH₂CL ; or
- R₂ = CH₃ and R₃ = CH₂CH₂CN ; or
- R₂ = CHF₂ and R₃ = CH₃; or
- R₂ = C₂H₅ and R₃ = CH₃ ; or
- 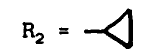 and R₃ = CH₃ ; or
- R₂ = CH₂OCH₃ and R₃ = CH₃; or

- R₂ = CH₂SCH₃ and R₃ = CH₃; or

- R₂ = CH₂ COO C₂H₅ and R₃ = CH₃; or
- 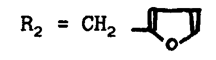 and R₃ = H ; or
- 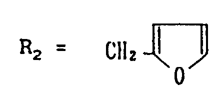 and R₃ = CH₃; or
- 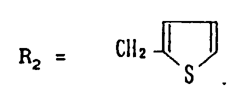 and R₃ = CH₃; or
- 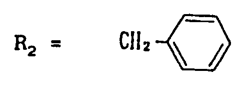 and R₃ = CH₃; or
- 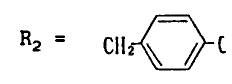 and R₃ = CH₃; or
- R₂ = CH₂CN and R₃ = CH₃; or
- 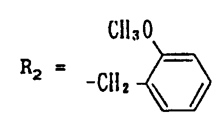 and R₃ = CH₃; or
- 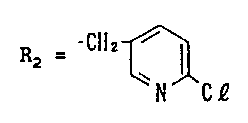 and R₃ = CH₃; or
- 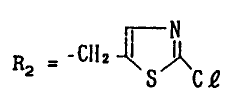 and R₃ = CH₃; or
- 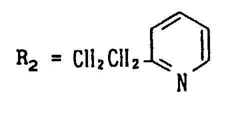 and R₃ = CH₃; or
- 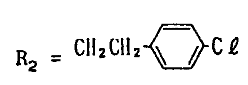 and R₃ = CH₃; or
- R₂ = OCH₃ and R₃ = CH₃.

### Best Mode for Carrying Out the Invention:

The compounds of this invention can be prepared in accordance with the following reaction schemes:

### (1) Preparation method 1 :

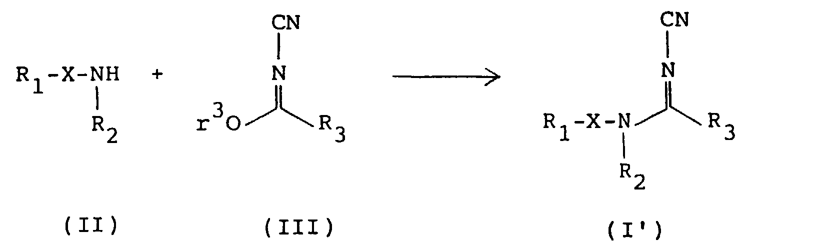

where r³ represents a C₁₋₅ alkyl:
and R₁, R₂, R₃ and X are as defined above. This reaction is carried out in an inactive organic solvent, preferably in an alcohol such as methanol, ethanol, between room temperature and the boiling point of the used solvent.

### (2) Preparation Method 2:

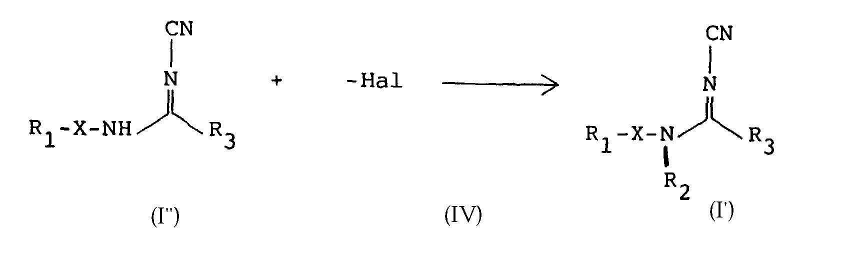

where Hal represents a halogen; and R₁, R₂, R₃ and X are as defined above.
This reaction is carried out in an inactive organic solvent, preferably DMF, THF, benzene acetonitrile, acetone, methylethylketone, in the presence of acid accepter such as potassium carbonate, NaH, triethylamine, between room temparature and the boiling point of the used solvent.

### (3) Preparation Method 3:

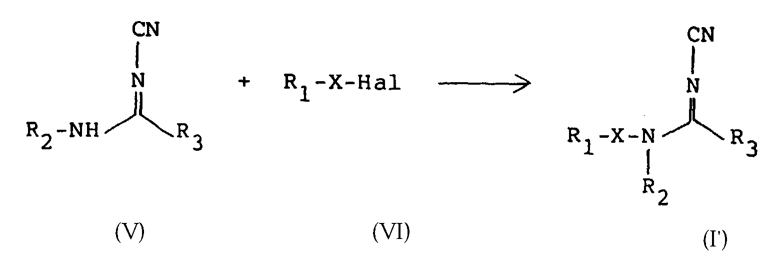

where R₁, R₂, R₃, X and Hal are as defined above. This reaction is carried out in the same manner as that of Preparation Method 2.

After the reaction is completed, an usual after-treatment gives the intended compound. The structure of the compounds of this invention was determined by such means as IR, NMR, MASS, etc. When R₂ is hydrogen in a compound of this invention, tautomers represented by 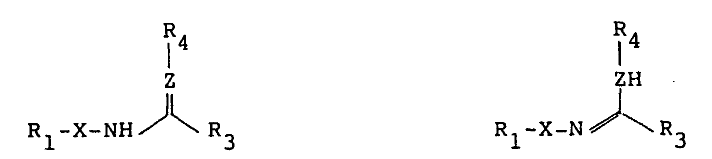 can exist.

The syn - aniti isomers, when Z represents N, as represented by, 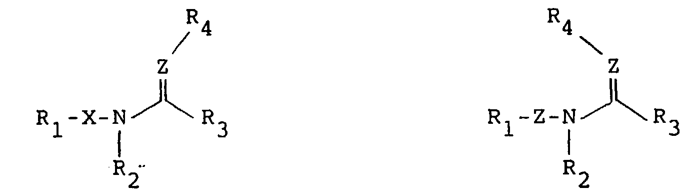 can also exist.

The ratio varies depending on e.g. conditions of instrumental analysis.

The following examples illustrate the present invention.

### Example 1:

N-cyano-N'-(2-chloro-5-pyridylmethyl)-N'-methylacetamidine: 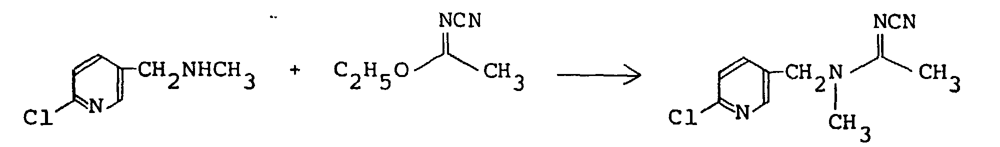 In 20ml of ethanol, 1.6g of N-methyl-2-cloro-5-pyridylmethylamine and 1.2g of ethyl-N-cyanoacetamidine were mixed and the mixture was stirred at room temperature over night. The solvent was then distilled off and the residue was purified by column chromatography on silica gel to afford 1.8g of compound No. 22, m.p. 101-103°C

### Example 2 :

N-cyano-N'-(2-chloro-5-pyridylmethyl)-N'-ethylacetamidine: 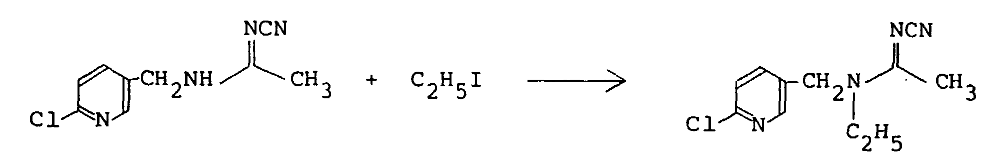 0.7g of sodium hydride (purity 60%) was added to the solution of 3.0g of N-cyano-N'-(2-chloro-5-pyridylmethyl)acetamidine in 20ml of N.N-dimthylformamide at ice bath temperature. After stirring it at the same temperature for 1 hour, 2.7g of ethyl iodide was added to the mixture, followed by stirring for 5 hours at room temperature. The reaction mixture was then poured into ice-water, extracted with ethyl acetate, dried over anhydrous magnesium sulfate and concentrated under reduced pressre. The residue obtained was purified by column chromatography on silica gel to afford 1.6g of compound No. 51. m.p. 100-101°C

### Example 3 :

N-cyano-N-(2-chloro-5-pyridylmethyl)-N'-methylacetamidine: 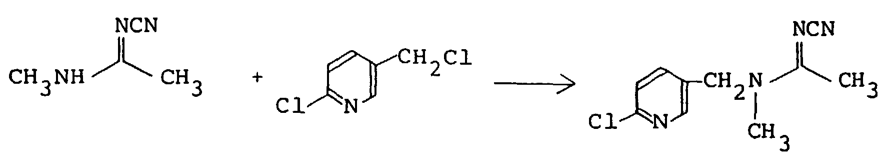 0.6g of sodium hydride (purity 60%) was added to the solution of 1.3g of N-cyano-N'-methylacetamidine in 20ml of N,N-dimethylformamide at ice bath temperature. After stirring it at the same temperature for 1 hour, 2.2g of 2-chloro-5-pyridylmethylchlride was added to the mixture, followed by stirring for 5 hours at room temperature. The reaction mixture was then poured into ice-water, extrated with ethyl acetate, dried over anhydrous magnesium sulfate and concentrated under reduced pressure. The residue obtained was purified by column chromatography on silica gel to afford 1.5g of compound No.22 m.p. 101-103°C

### Reference Example :

N-(2-chloro-5-pyridylmethyl)-N-methylacetamidine hydrochloride :

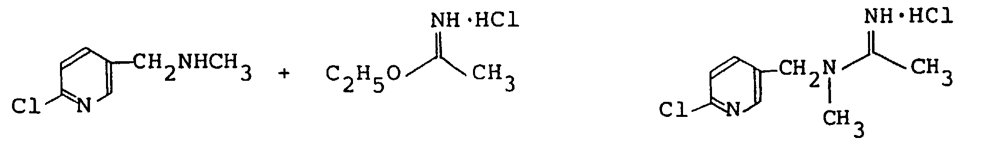

To 40ml of ethanol was added 5.lg of N-(2-chloro-5-pyridylmethyl)-N-Methylamine and then 4g of ethyl acetimidate hydrochloride at 0°C. After stirring for an hour, the reaction mixture was allowed to warm to room temperature and stirred over night. The solvent was then distilled off. The obtained white residue was washed with diethyl ether to afford 7.3g of the title compound m.p. 192-197°C

Typical examples of this invention including those described above are listed in Table 1.

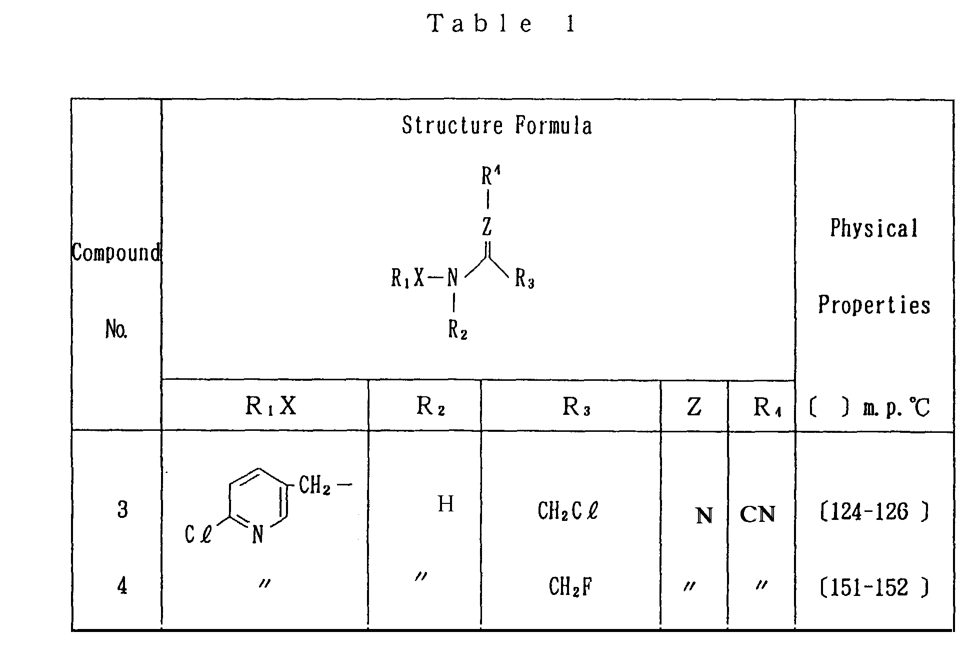

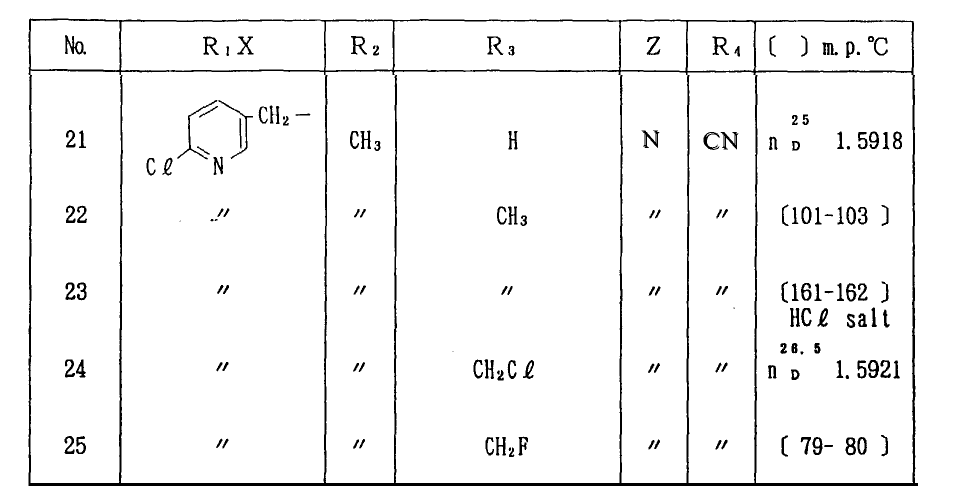

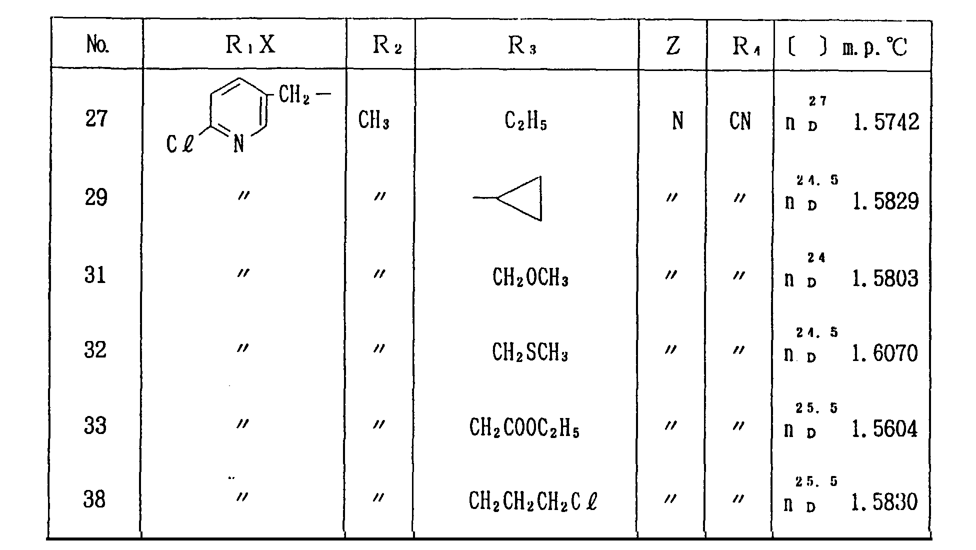

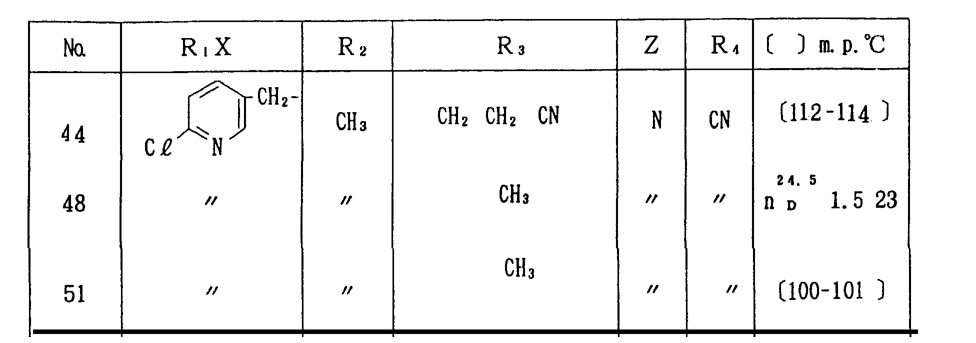

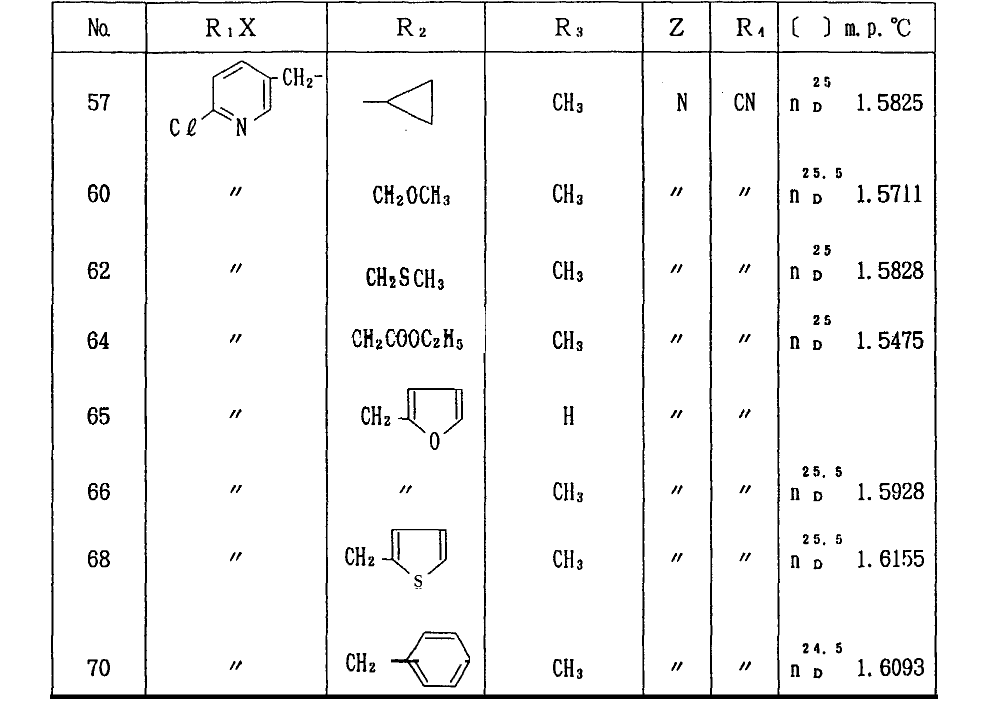

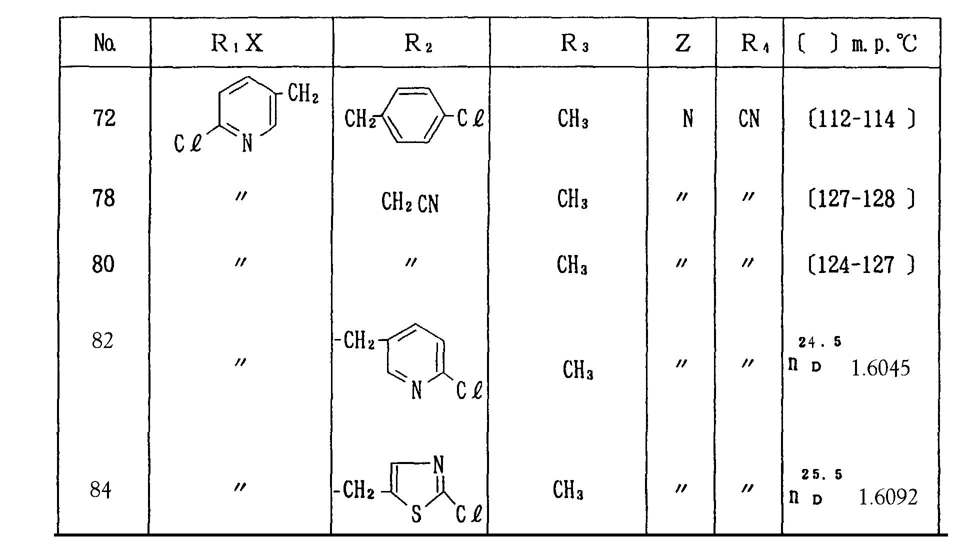

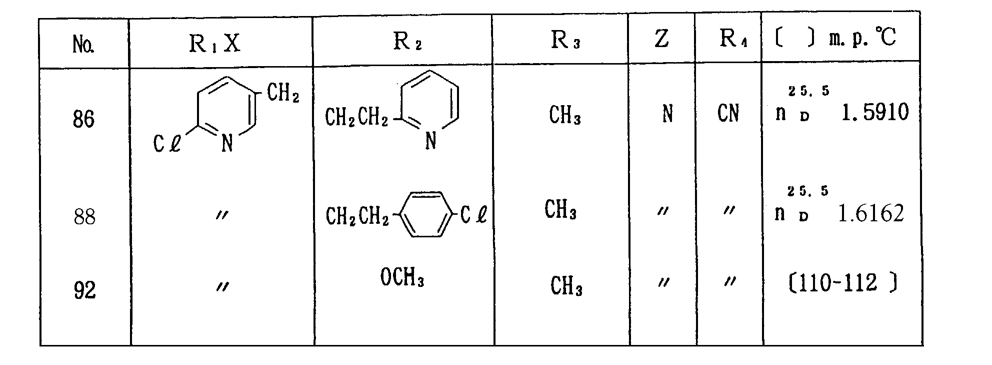

The compounds of this invention exhibit high insecticidal activities against various species of insect pests such as cutworms, diamondback moth, aphids, leafhoppers and planthoppers. In recent years the decrease of the control effects of organophosphorus and carbamate insecticides, which is caused by the development of resistance to these insecticides, has become a serious problem. In such situations, the development of new insecticides which is effective on the resistant pests has been desired. The compounds of this invention possess superior insecticidal activities against not only susceptible strains but also resistant ones.

The insecticides covered by this invention contain as active ingredients one or more types of the compounds as expressed by the general formula (1). These active ingredients, may be used as-produced but normally they are used in any of the forms which ordinary agricultural chemicals can take, namely wettable powder, dust, emulsifiable concentrate, suspension concentrates, smoking chemicals, fumigant, granule, or other formulations. For additives and carriers are used soybean flour, wheat flour or other vegetable flours, diatomaceous earth, apatite, gypsum, talc, pyrophyllite, clay or other fine mineral powders, when solid formulations are intended.

When liquid formulations are intended, then for solvents are used kerosene, mineral oil, petroleum, solvent naphtha, xylene, cyclohexane, cyclohexanone, dimethylformamide, dimethylsulfoxide, alcohol, acetone, water, etc. A surface active agent may, if necessary, be added in order to give a homogeneous and suitable formulation. The wettable powders, emulsifiable concentrates, suspension concentrates, etc. thus obtained are diluted with water into suspensions or emulsions of a prescribed concentration, before they are actually sprayed on plants in the field. In the case of dusts or granules, they are directly applied without further processing.

It goes without saying that the compound(s) of this invention is effective even alone, but it can be used by mixing with various types of insecticides, acaricides and fungicides.

Typical examples of acaricides and insecticides which can be used by mixing with the compounds of this invention are described below:
Acaricides (fungicides):
chlorobenzilate, chloropropylate, proclonol, bromopropylate, dicofol, dinobuton, binapacryl, chlordimeform, amitraz, propargite, PPPS, benzoximate, hexythiazox, fenbutatin oxide, polynactine, chinomethionat, thioquinox, chlorfenson, tetradifon, phenproxide, avermectins, clofentezine, flubenzimine, fenazaquin, pyridaben, fenproximate, chlorfenethol, thiophanate-methyl, benomyl, thiram, iprobenfos, edifenfos, fthalide, probenazole, isoprothiolane, chorothalonil, captan, polyoxin-B, blasticidin-S, kasugamycin, validamycin, tricyclazole, pyroquilon, phenazine oxide, mepronil, flutolanil, pencycuron, iprodione, hymexazole, metalaxyl, triflumizole, diclomezine, tecloftalam, vinclozolin, procymidone, bitertanol, triadimefon, prochloraz, pyrifenox, fenarimal, fenpropimorph, triforine, metalaxyl, oxycarboxin, pefrazoate, diclomedine, fluazinam, oxadixyl, ethoquinolac, TPTH, propamocarb, fosetyl, dihydrostreptomycin, anilazine, dithianon, diethofencarb. Organophosphorus-type and carbamate-type insecticides(acaridides):
fenthion, fenitrothion, diazinon, chlorpyrifos, oxydeprofos, vamidothion, phenthoate, dimethoate, formothion, malathion, trichlorfon, thiometon, phosmet, menazon, dichlorvos, acephate, EPBP, dialifos, parathion-methyl, oxydemeton-methyl, ethion, aldicarb, propoxur, methomyl, fenobucarb, BPMC, pyraclofos, monocrotophos, salithion, cartap, carbosulfan carbofuran, benfuracarb, metolcarb, carbaryl, pirimicarb, ethiofencarb, fenoxycarb,
Pyrethroide-type insecticides (acaricides ):
permethrin, cypermethrin, deltamethrin, fenvalerate, fenpropathrin, pyrethrins, allethrin, tetramethrin, resmethrin, parthrin, dimethrin, proparthrin, bifenthrin, prothrin, fluvalinate, cyfluthrin, cyhalothrin, flucythrinate, ethofenprox, cycloprothrin, tralomethrin, silaneophan.
Benzoylphenylurea-type and other types insecticides:
diflubenzuron, chlorfluazuron, triflumuron, teflubenzuron, buprofezin, pyriproxyfen, flufenoxuron, Machine oil.

Same examples of the formulations are given below. The carriers, surface-active agents, etc. that are added, however, are not limited to these Examples.

| Example 4: Emulsifiable concentrate | |
|---|---|
| A compound of this invention | 10 parts |
| Alkylphenyl polyoxyethylene | 5 parts |
| Dimethyl formamide | 50 parts |
| Xylene | 35 parts |

These components are mixed and dissolved and, for use in spraying, the liquid mixture is water-diluted into an emulsion.

| Example 5 : Wettable powder | |
|---|---|
| A compound of this invention | 20 parts |
| Higher alcohol sulfuric ester | 5 parts |
| Diatomaceous earth | 70 parts |
| Silica | 5 parts |

These components are mixed and ground to fine powder, which for use in spraying, are water-diluted into a suspension.

| Example 6 : Dust | |
|---|---|
| A compound of this invention | 5 parts |
| Talc | 94.7 parts |
| Silica | 0.3 parts |

These are mixed and ground and used as-ground in spraying.

| Example 7 : Granule | |
|---|---|
| A compound of this invention | 5 parts |
| Clay | 73 parts |
| Bentonite | 20 parats |
| Sodium dioctylsulfosuccinate | 1 part |
| Sodium phosphate | 1 part |

The above compounds are granulated, and applied as it is when used.

### Industrial applicability:

The tests below show the insecticidal activity of the compounds of this invention.

### Test 1 Efficacy for cotton aphid

30 to 50 insects of cotton aphid per plot were inoculated using a small brush on cucumber leaves which were seeded in pots, 10cm in diameter, and 10 days old after germination. A day later, wounded insect pests were removed, and a chemical solution, which was prepared in the way that the emulsifiable concentrate described in Example 7 of the above example of insecticide was diluted with water to 125 ppm of compound concentration according to the prescription, was sprayed. The pots were placed in a thermostatic room at temperature of 25°C and humidity of 65%. The number of survival pests was counted 7 days later and the control efficacy was calculated by comparing with that of untreated plot. The results are shown in Table 2.

**Table 2**

| | Control Efficacy (7 days later) |
|---|---|
| Compound No. | 125 ppm |
| 3 | 100 % |
| 4 | 100 |
| 21 | 100 |
| 22 | 100 |
| 23 | 100 |
| 24 | 100 |
| 25 | 100 |
| 27 | 100 |
| 29 | 100 |
| 31 | 100 |
| 32 | 100 |
| 33 | 100 |
| 38 | 100 |
| 44 | 100 |
| 48 | 100 |
| 57 | 100 |
| 60 | 100 |
| 62 | 100 |
| 64 | 100 |
| 66 | 100 |
| 68 | 100 |
| 70 | 100 |
| 72 | 100 |

| Compound No. | Control Efficacy |
|---|---|
| 78 | 100 |
| 80 | 100 |
| 82 | 100 |
| 84 | 100 |
| 86 | 100 |
| 88 | 100 |
| 92 | 100 |
| Comparative compound A | 27 |
| " B | 100 |

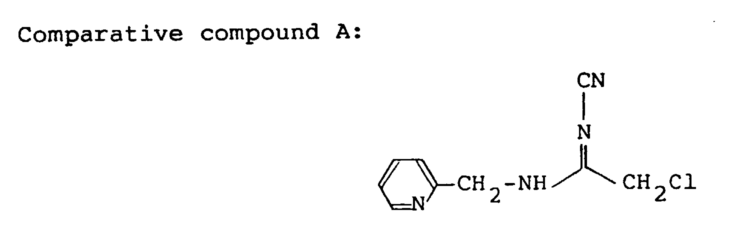

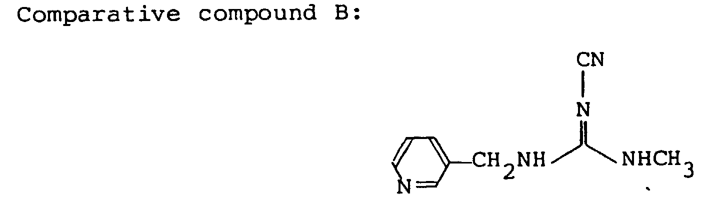

### Test 2 Efficacy for green rice leafhopper

Rice seedlings of 7 days old after germination were immersed in a chemical solution, which was prepared in the way that the emulsifiable concentrate described in Example 7 of the above example of insecticide was diluted with water to 125 ppm of compound concentration according to prescription, for 30 seconds. After dried in air, the treated seedlings were placed in test tubes and 10 insects of 3rd-instar larvae of green rice leafhopper resistant to the organophosphorus and carbamate insecticides were inoculated. The tubes were covered with gauze, and placed in a thermostatic room at temperature of 25°C and humidity of 65%. The mortality was checked 5 days later.

The results are shown in Table 3.

**Table 3**

| | % mortality (5 days later) |
|---|---|
| Compound No. | 125 ppm |
| 4 | 100% |
| 21 | 100 |
| 22 | 100 |
| 23 | 100 |
| 24 | 100 |
| 25 | 100 |
| 27 | 100 |
| 29 | 100 |
| 31 | 100 |
| 32 | 100 |
| 33 | 100 |
| 44 | 100 |
| 48 | 100 |
| 51 | 100 |
| 57 | 100 |
| 60 | 100 |
| 62 | 100 |
| 66 | 100 |
| 68 | 100 |
| 72 | 100 |

| Compound No. | % Mortality |
|---|---|
| 78 | 100 |
| 82 | 100 |
| 84 | 100 |
| 86 | 100 |
| 88 | 100 |
| 92 | 100 |
| Comparative Compound A | 0 |
| " B | 0 |
| " C | 0 |

Comparative compound A and B: The same as test 1

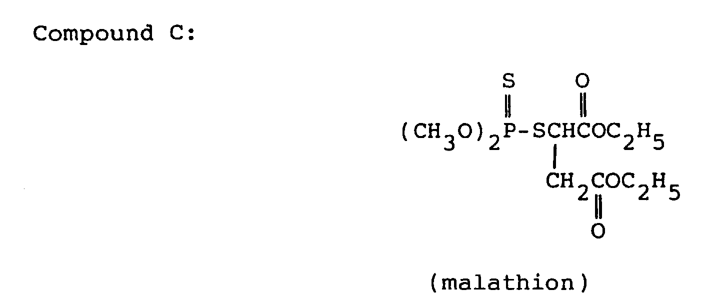

### Test 3 Efficacy for rice armyworm

The test compounds were formulated into the wettable powder in the same manner as Example 5. The compounds were diluted with water to 125 ppm. A maize leaf was immersed in the chemical solution for 30 seconds. After air-dried, the treated leaf was placed in a petri dish and five 3rd-instar larvae of rice armyworm were inoculated. The petri dishes were covered with glass lids, and placed in a thermostatic room at 25°C and 65% relative humidity. The mortality was checked 5 days later. Two replications were conducted in the each test. The results are shown in Table 4.

**Table 4**

| | % mortality (5 days later) |
|---|---|
| Compound No. | 125 ppm |
| 21 | 100 % |
| 22 | 100 |
| 23 | 100 |
| 24 | 100 |
| 25 | 100 |
| 51 | 100 |
| 57 | 100 |
| 88 | 100 |
| 92 | 100 |
| Comparative compound A | 0 |
| " B | 0 |
| " D | 40 |

Comparative compound A and B: The same as Test 1

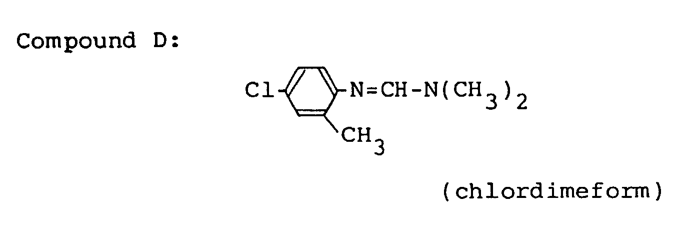

## Claims

1. Compound of the formula 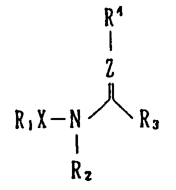 wherein in each case 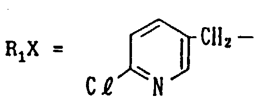 Z = N and R₄ = CN and wherein
- R₂ = H and R₃ = CH₂ CL ; or
- R₂ = H and R₃ = CH₂F; or
- R₂ = CH₃ and R₃ = H ; or
- R₂ = CH₃ and R₃ = CH₃ ; or
- R₂ = CH₃ and R₃ = CH₃ HCL salt ; or
- R₂ = CH₃ and R₃ = CH₂CL; or
- R₂ = CH₃ and R₃ = CH₂F; or
- R₂ = CH₃ and R₃ = C₂H₅ ; or
- R₂ = CH₃ and 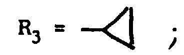 or
- R₂ = CH₃ and R₃ = CH₂OCH₃ or;
- R₂ = CH₃ and R₃ - CH₂SCH₃; or
- R₂ = CH₃ and R₃ = CH₂ COOC₂H₅
- R₂ = CH₃ and R₃ = CH₂CH₂CH₂CL ; or
- R₂ = CH₃ and R₃ = CH₂ CH₂ CN ; or
- R₂ = CHF₂ and R₃ = CH₃; or
- R₂ = C₂H₅ and R₃ = CH₃ ; or
- 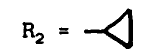 and R₃ = CH₃ ; or
- R₂ = CH₂OCH₃ and R₃ = CH₃; or
- R₂ = CH₂SCH₃ and R₃ = CH₃; or
- R₂ = CH₂ COO C₂H₅ and R₃ = CH₃; or
- 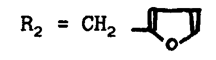 and R₃ = H ; or
- 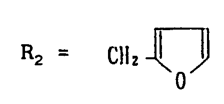 and R₃ = CH₃; or
- 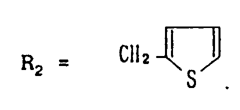 and R₃ = CH₃; or
- 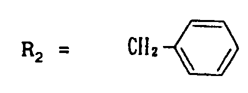 and R₃ = CH₃; or
- 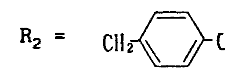 and R₃ = CH₃; or
- R₂ = CH₂CN and R₃ = CH₃; or
- 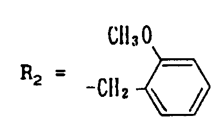 and R₃ = CH₃; or
- 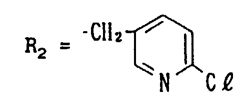 and R₃ = CH₃; or 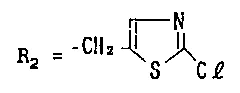 and R₃ = CH₃; or
- 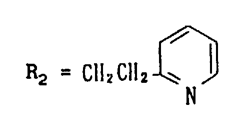 and R₃ = CH₃; or
- 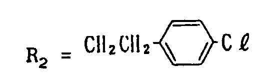 and R₃ = CH₃ ; or
- R₂ = OCH₃ and R₃ = CH₃.

2. An insecticidal composition comprising a compound according to claim 1 as an active ingredient.

3. A process for the preparation of a compound having the formula 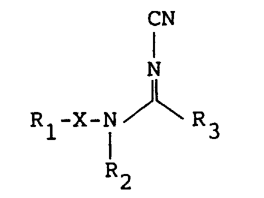 which comprises reacting a compound having the formula 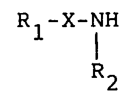 with a compound having the formula 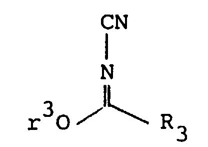 wherein r³ represents a C₁₋₅ alkyl; and R₁, R₂, R₃ and X are as defined above.

4. A process for the preparation of a compound having the formula 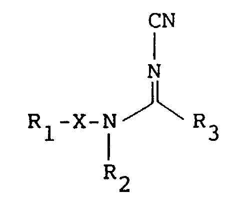 which comprises reacting a compound having the formula 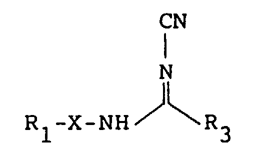 with a compound having the formula
R₂-Hal
wherein Hal represents a halogen atom; and R₁, R₂, R₃ and X are as defined above.

5. A process for the preparation of a compound having the formula 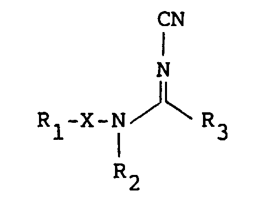 which comprises reacting a compound having the formula 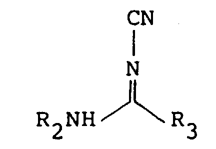 with a compound having the formula
R₁-X-Hal
wherein R₁, R₂, R₃, X and Hal are as defind above.

## Patentansprüche

1. Verbindung der Formel 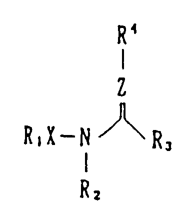 in der in jedem Falle 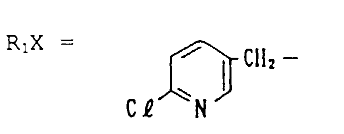 Z = N und R₄ = CN und in der
- R₂ = H und R₃ = CH₂Cl oder
- R₂ = H und R₃ = CH₂F oder
- R₂ = CH₃ und R₃ = H oder
- R₂ = CH₃ und R₃ = CH₃ oder
- R₂ = CH₃ und R₃ = CH₃, HCl-Salz, oder
- R₂ = CH₃ und R₃ = CH₂Cl oder
- R₂ = CH₃ und R₃ = CH₂F oder
- R₂ = CH₃ und R₃ = C₂H₅ oder
- R₂ = CH₃ und 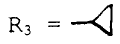 oder
- R₂ = CH₃ und R₃ = CH₂OCH₃ oder
- R₂ = CH₃ und R₃ = CH₂SCH₃ oder
- R₂ = CH₃ und R₃ = CH₂COOC₂H₅,
- R₂ = CH₃ und R₃ = CH₂CH₂CH₂Cl oder
- R₂ = CH₃ und R₃ = CH₂CH₂CN oder
- R₂ = CHF₂ und R₃ = CH₃ oder
- R₂ = C₂H₅ und R₃ = CH₃ oder 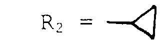 und R₃ = CH₃ oder
- R₂ = CH₂OCH₃ und R₃ = CH₃ oder
- R₂ = CH₂SCH₃ und R₃ = CH₃ oder
- R₂ = CH₂COOC₂H₅ und R₃ = CH₃ oder 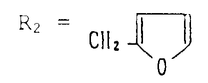 und R₃ = H oder
- 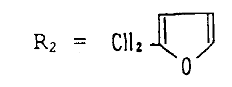 und R₃ = CH₃ oder
- 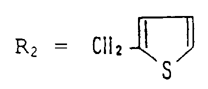 und R₃ = CH₃ oder
- 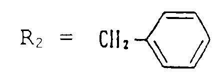 und R₃ = CH₃ oder
- 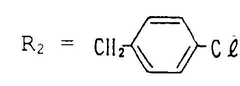 und R₃ = CH₃ oder
- R₂ = CH₂CN und R₃ = CH₃ oder
- 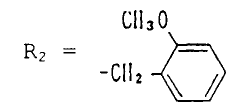 und R₃ = CH₃ oder
- 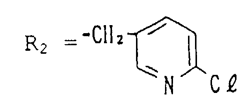 und R₃ = CH₃ oder
- 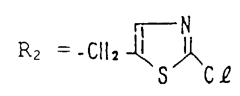 und R₃ = CH₃ oder
- 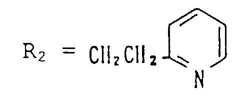 und R₃ = CH₃ oder
- 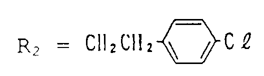 und R₃ = CH₃ oder
- R₂ = OCH₃ und R₃ = CH₃.

2. Insektizide Zusammensetzung, umfassend als Aktivsubstanz eine Verbindung nach Anspruch 1.

3. Verfahren zur Herstellung einer Verbindung der Formel 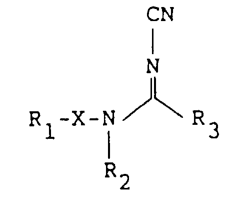 welches umfaßt, eine Verbindung der Formel 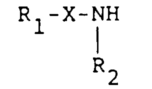 mit einer Verbindung der Formel 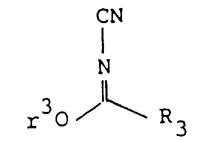 in der r³ für ein C₁₋₅-Alkyl steht, und wobei R₁, R₂, R₃ und X wie vorstehend definiert sind, umzusetzen.

4. Verfahren zur Herstellung einer Verbindung der Formel 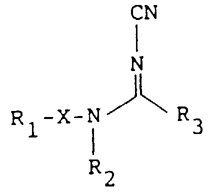 welches umfaßt, eine Verbindung der Formel 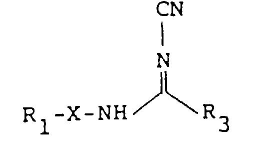 mit einer Verbindung der Formel
R₂-Hal
in der Hal für ein Halogenatom steht, und wobei R₁, R₂, R₃ und X wie vorstehend definiert sind, umzusetzen.

5. Verfahren zur Herstellung einer Verbindung der Formel 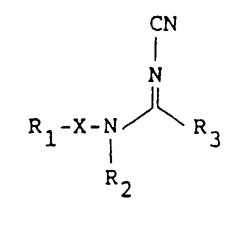 welches umfaßt, eine Verbindung der Formel 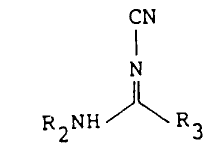 mit einer Verbindung der Formel
R₁-X-Hal
wobei R₁, R₂, R₃, X und Hal wie vorstehend definiert sind, umzusetzen.

## Revendications

1. Composé de formule 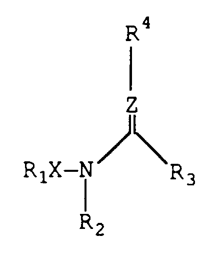 où dans chaque cas, 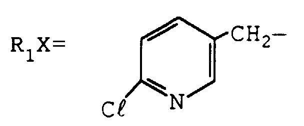 Z = N et R₄ = CN et où
- R₂ = H et R₃ = CH₂Cℓ ; ou
- R₂ = H et R₃ = CH₂F ; ou
- R₂ = CH₃ et R₃ = H ; ou
- R₂ = CH₃ et R₃ = CH₃ ; ou
- R₂ = CH₃ et R₃ = CH₃ sel HCℓ ; ou
- R₂ = CH₃ et R₃ = CH₂Cℓ ; ou
- R₂ = CH₃ et R₃ = CH₂F ; ou
- R₂ = CH₃ et R₃ = C₂H₅ ; ou
- R₂ = CH₃ et 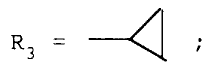 ou
- R₂ = CH₃ et R₃ = CH₂OCH₃ ; ou
- R₂ = CH₃ et R₃ - CH₂SCH₃ ; ou
- R₂ = CH₃ et R₃ = CH₂COOC₂H₅
- R₂ = CH₃ et R₃ = CH₂CH₂CH₂Cℓ ; ou
- R₂ = CH₃ et R₃ = CH₂CH₂CN ; ou
- R₂ = CHF₂ et R₃ = CH₃ ; ou
- R₂ = C₂H₅ et R₃ = CH₃ ; ou 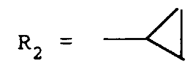
et R₃ = CH₃; ou
R₂ = CH₂OCH₃ et R₃ = CH₃ ; ou
- R₂ = CH₂SCH₃ et R₃ = CH₃ ; ou
- R₂ = CH₂ COO C₂H₅ et R₃ = CH₃ ; ou
- 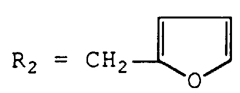 et R₃ = H ; ou
- 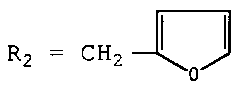 et R₃ = CH₃ ; ou
- 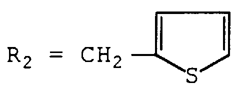 et R₃ = CH₃; ou
- 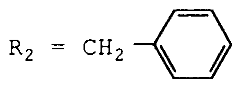 et R₃ = CH₃ ; ou
- 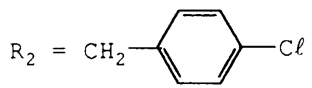 et R₃ = CH₃ ; ou
- R₂ = CH₂CN et R₃ = CH₃ ; ou
- 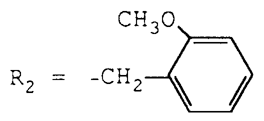 et R₃ = CH₃; ou
- 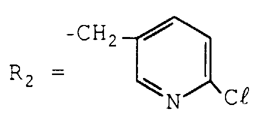 et R₃ = CH₃ ; ou
- 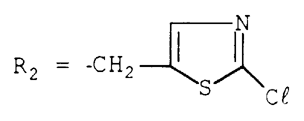 et R₃ = CH₃ ; ou
- 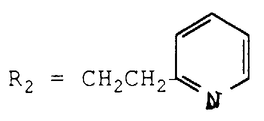 et R₃ = CH₃; ou
- 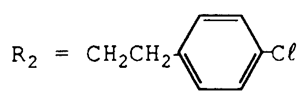 et R₃ = CH₃; ou
- R₂ = OCH₃ et R₃ = CH₃

2. Composition insecticide comprenant un composé selon la revendication 1 en tant qu'ingrédient actif.

3. Procédé pour la préparation d'un composé ayant la formule 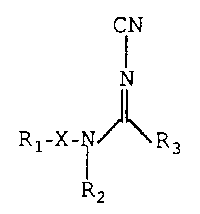 qui comprend la réaction d'un composé ayant la formule 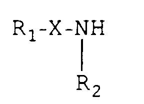 avec un composé ayant la formule 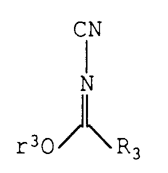 où r³ représente un alkyle en C₁₋₅ ; et R₁, R₂, R₃ et X sont tels que définis ci-dessus.

4. Procédé pour la préparation d'un composé ayant la formule 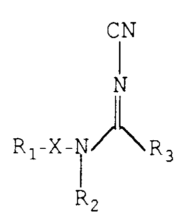 qui comprend la réaction d'un composé ayant la formule 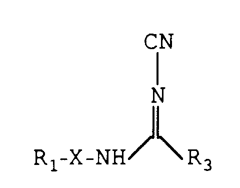 avec un composé ayant la formule
R₂-Hal
où Hal représente un atome d'halogène ; et R₁, R₂, R₃ et X sont tels que définis ci-dessus.

5. Procédé pour la préparation d'un composé ayant la formule 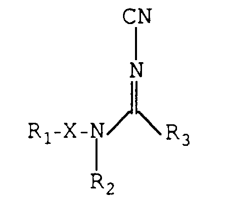 qui comprend la réaction d'un composé ayant la formule 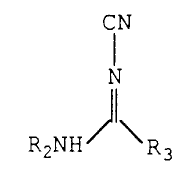 avec un composé ayant la formule
R₁-X-Hal
où R₁, R₂, R₃, X et Hal sont tels que définis ci-dessus.
